# EUROPÄISCHE PATENTSCHRIFT

(11) **EP 1 137 643 B1**
(45) Veröffentlichungstag und Bekanntmachung des Hinweises auf die Patenterteilung: **18.12.2002**
(21) Anmeldenummer: 99961031.4
(22) Anmeldetag: 01.12.1999
(51) Int. Cl.: C07D 301/12, C07D 303/04

(54) **INTEGRIERTES VERFAHREN ZUR HERSTELLUNG VON EPOXIDEN AUS OLEFINEN**
INTEGRATED METHOD FOR PRODUCING EPOXIDES FROM OLEFINS
PROCEDE INTEGRE POUR LA PRODUCTION DES EPOXIDES A PARTIR DES OLEFINES

(30) Priorität: 11.12.1998 DE 19857137
(43) Veröffentlichungstag der Anmeldung: 04.10.2001
(73) Patentinhaber: Bayer Aktiengesellschaft, 51368 Leverkusen (DE)
(72) Erfinder: VOGTEL, Peter, D-51373 Leverkusen (DE); DORF, Ernst-Ulrich, D-47802 Krefeld (DE); WEGENER, Gerhard, D-40822 Mettmann (DE); WEISBECK, Markus, D-51465 Bergisch Gladbach (DE)
(86) Internationale Anmeldenummer: EP9909340
(87) Internationale Veröffentlichungsnummer: WO00035894

(56) Entgegenhaltungen:
- EP-A- 0 812 836
- DE-A- 19 723 950
- US-A- 5 523 426
- US-A- 5 599 955
- US-A- 5 599 956

## Beschreibung

Die Erfindung betrifft ein integriertes Verfahren zur Herstellung von epoxidierten Olefinen. In der ersten Stufe werden verdünnte Wasserstoffperoxidlösungen aus den Elementen Wasserstoff und Sauerstoff unter Katalyse hergestellt und in einer anschließenden Flüssigphasenepoxidation mit Olefin in Gegenwart von Titansilikalit zu epoxidiertem Olefin umgesetzt und die Lösungsmittel wieder in den H₂O₂-Prozeß zurückgeführt.

Propenoxid stellt eine der wichtigsten Grundchemikalien der Chemischen Industrie dar. Das Einsatzgebiet liegt mit einem Anteil von über 60 % im Kunststoffsektor, speziell zur Herstellung von Polyetherpolyolen für die Synthese von Polyurethanen. Daneben werden noch größere Marktanteile im Bereich der Glykole, besonders bei den Schmier- und Frostschutzmitteln, von den Propenoxid-Derivaten belegt.

Die Herstellung von epoxidierten Olefinen ist prinzipiell bekannt. Es wird hierbei von einem Olefin ausgegangen, welches in verschiedenster Art oxidiert wird. Aus ökologischen Überlegungen ist jedoch die Oxidation mit Wasserstoffperoxid oder Luft zu bevorzugen.

Die Titansilikalit-katalysierte Epoxidation mit reinem Sauerstoff als Oxidationsmittel gelingt in Anwesenheit eines Redoxsystems, bestehend aus Alkylanthrahydrochinon und Alkylanthrachinon (US 5 221 795). Nachteil dieser Reaktion ist der ständige Verlust von kleinen Mengen Anthrachinon und organischen Lösungsmittel infolge oxidativer Zersetzung dieser Organika.

An platinmetallhaltigen Titansilikaliten gelingt die Propenoxidation mit geringer Ausbeute (ca. 1 bis 2 %) und ungenügenden Propenoxid-Selektivitäten von 60 bis 70 % mit einem Gasgemisch bestehend aus molekularem Sauerstoff und molekularem Wasserstoff (WO-97/47 386, WO-96/02323). Als Nebenreaktion auftretende Hydrierungen führen zu großen Mengen Propan als Nebenprodukt.

In den Patenten US-5 623 090 und WO-98/00 413-15 wird eine Direktoxidation von Propen zu Propenoxid mit molekularem Sauerstoff in Gegenwart von Wasserstoff offenbart. Als Katalysator wird handelsübliches Titandioxid verwendet, das mit fein dispergierten Goldteilchen belegt ist. Diese Verfahren haben neben geringen Ausbeuten alle den Nachteil, daß sie durch den Goldgehalt des Katalysators sehr teuer sind. Für eine wirtschaftliche Nutzung ist daher die Entwicklung von Katalysatoren mit deutlich besseren Katalysatoraktivitäten bei stark erhöhter Katalysatorstandzeit unbedingt weiter notwendig.

US 4 833 260 beschreibt Titansilikalit-Katalysatoren, die effektiv die Epoxidation von Olefinen mit dem Oxidationsmittel Wasserstoffperoxid in der Flüssigphase ermöglichen. Bei den Silikaliten ist ein kleiner Teil des Gitter-Siliziums durch Titan ausgetauscht (US 4 410 501). Die hohen Kosten für Wasserstoffperoxid als Oxidationsmittel stehen allerdings bisher einer großtechnischen Anwendung entgegen. Ein Großteil der Kosten bei Verwendung von Wasserstoffperoxid entstehen durch das Wasserstoffperoxid selbst, da die Konzentrationen an Wasserstoff und Sauerstoff während der Herstellung aus Sicherheitsgründen sehr genau beachtet werden müssen. Überwiegend werden Reaktionslösungen mit geringen H₂O₂-Konzentrationen erhalten, die dann aufwendig aufkonzentriert, gereinigt und stabilisiert werden müssen.

Die Herstellung von Wasserstoffperoxid ist prinzipiell Stand der Technik.

Es ist seit langem bekannt, daß Wasserstoffperoxid auch direkt aus den Elementen Wasserstoff und Sauerstoff an geeigneten Katalysatoren hergestellt werden kann.

Es ist ebenfalls seit langem bekannt, daß Mischungen aus gasförmigem Sauerstoff und Wasserstoff explosive Gasmischungen ergeben. Aus diesem Grunde arbeiten alle industriellen H₂O₂-Verfahren mit einer indirekten Kombination von Wasserstoff und Sauerstoff.

Über 90 % der Wasserstoffperoxid-Weltproduktion erfolgt zur Zeit nach dem Anthrachinon-Verfahren bei denen typischerweise Alkylanthrachinone als chemische Hilfsstoffe eingesetzt werden. Nachteil dieser Reaktion ist der ständige Verlust von kleinen Mengen Anthrachinon und organischen Lösungsmitteln infolge der Oxidation sowie von thermischer Zersetzung dieser Organika und kostenintensive Extraktions-, Reinigungs- und Destillationsschritte.

Die Direktsynthese von H₂O₂ aus den Elementen Wasserstoff und Sauerstoff ist Gegenstand intensiver Forschungsbemühungen, hat bislang jedoch zu keiner kommerziellen Verwendung geführt. Neben den sicherheitstechnischen Problemen ist das vorrangige Problem, die nachfolgende Zersetzung des entstandenen Wasserstoffperoxid zu Wasser und Sauerstoff zu verhindern. Dieses Problem wird mit kontinuierlichen Verfahren gelöst, die mit hohen Durchflußgeschwindigkeiten arbeiten. Dies hat jedoch zur Folge, daß bei niedrigen Reaktionsgeschwindigkeiten die im Austrag enthaltenen Wasserstoffperoxidkonzentrationen für eine wirtschaftliche Nutzung dieses Verfahrens zu gering werden.

Die US 4 009 252 offenbart für die Bildung von Wasserstoffperoxid aus Wasserstoff und Sauerstoff an palladiumhaltigen Katalysatoren ein optimales Verhältnis von O₂ zu H₂ im Bereich 1,5 : 1 bis 20 : 1, also im Explosionsbereich.

Als katalytisch aktive Spezies werden am häufigsten die Übergangsmetalle der 8. Nebengruppe, meistens Palladium, oder Platin verwendet. Das Edelmetall kann auf verschiedenen Trägern, wie TiO₂, SiO₂, Al₂O₃, Teflon, Aktivkohle oder Katalysatormonolithen, die aus Geweben, wie z.B. V4a, aufgebaut sind, aufgebracht werden, wobei Aktivkohle die häufigste Verwendung findet. Verfahren auf der Basis derartiger katalytischer Systeme wurden von zahlreichen Firmen und Institutionen patentiert, wie z. B. US 4 279 883, US 4 661 337, EP-117 306 und DE-196 42 770.

Die US 4 336 238 und US 4 336 239 beschreiben die Umsetzung von Wasserstoff und Sauerstoff zu Wasserstoffperoxid an palladiumhaltigen Katalysatoren in organischen Lösungsmitteln oder Lösungsmittelgemischen, die gegebenenfalls auch Wasser enthalten. Die erhaltenen Wasserstoffperoxidkonzentrationen von maximal 2,4 Gew.-% bei Verwendung von Reaktionsgasmischungen, die weniger als 5 Vol.-% Wasserstoff enthalten, sind für eine wirtschaftliche Anwendung zu gering. Nach 285 Stunden Betriebsdauer ist die Katalysatoraktivität zudem auf 69 % des ursprünglichen Wertes gesunken., was für eine industrielle Anwendung immer noch zu gering ist.

Die US 5 352 645 und WO-92/04 976 beschreiben spezielle feste Träger aus sprühgetrocknetem kolloidalen Kieselgel. EP- 627 381 offenbart die Verwendung von Niob-, Tantal-, Molybdän oder Wolframoxiden als Trägermaterialien, die sich durch hohe Säurefestigkeit auszeichnen.

Die Herstellung des Wasserstoffperoxides erfolgt in den genannten Schriften jedoch immer nach Batch- oder halbkontinuierlichen Verfahren, die für eine industrielle Nutzung wenig geeignet sind. Zudem lassen die kurzen Reaktionszeiten keine Aussage über die Lebensdauer der Katalysatoren zu.

DE-A-196 42 770 offenbart die Wasserstoffperoxidherstellung an palladiumhaltigen Katalysatormonolithen, z.B. mit Palladium imprägnierten V4a Netzen bzw. Geweben. Als Lösungsmittel werden C₁-C₃-Alkohole, bzw. Mischungen mit Wasser eingesetzt. Als katalytisch aktive Komponente wird vornehmlich Palladium eingesetzt; als Promotorsubstanzen sind vorzugsweise Edelmetalle, wie Platin, Rhodium, Gold und Silber geeignet.

Für konventionelle Anlagen von H₂O₂ haben sich alle diese Verfahren wegen der geringen H₂O₂-Konzentration sowie teilweise die Anwesenheit von Lösungsmittel nicht durchgesetzt.

Bis heute ist kein einziges Verfahren zur Herstellung von Propylenoxid aus Propylen und Wasserstoffperoxid bekannt, bei dem direkt nicht-aufkonzentrierte und nur grob vorgereinigte Wasserstoffperoxid Lösungen eingesetzt werden und diese nach der Propylenoxidation wieder in die Wasserstoffperoxidherstellung zurückgeführt werden.

Überraschenderweise haben wir gefunden, daß sich nicht-aufkonzentrierte und nur grob gereiningte Wasserstoffperoxid-Lösungen direkt aus der Herstellung zur Herstellung von epoxidierten Olefinen, insbesondere Propenoxid durch die katalysierte Epoxidation von Olefinen mittels H₂O₂ in Gegenwart eines synthetischen Titan-Atome enthaltenden Zeoliths der allgemcinen Formel xTiO₂·(1-x)SiO₂, wobei x im Bereich von 0,0001 bis 0,04 liegt einsetzen lassen.

Die in der ersten Stufe hergestellten wäßrigen bzw. wäßrig-alkoholischen Wasserstoffperoxidlösungen können nach geringfügiger Reinigung selektiv mit Olefinen in Gegenwart besagter synthetischer Titan-Atome enthaltender Zeolithe zum epoxidierten Olefin umgesetzt werden. Das Verfahren sieht die Trennung und Rückführung von Lösungsmitteln vor, so daß diese ohne zusätzliche Reinigung wieder in den Wasserstoffperoxid-Herstellprozeß zurückgeführt werden können.

Die vorliegende Erfindung betrifft somit ein Verfahren zur Herstellung von epoxidierten Olefinen aus Olefinen und alkoholischen oder wäßrig-alkoholischen verdünnten Wasserstoffperoxidlösungen in Gegenwart eines synthetischen Titan-Atome enthaltenden Zeoliths der allgemeinen Formel xTiO₂·(1-x)SiO₂, wobei x im Bereich von 0,0001 bis 0,04 liegt, dadurch gekennzeichnet, daß das Verfahren die Schritte
a) Herstellung der alkoholischen oder wäßrig-alkoholischen verdünnten Wasserstoffperoxidlösungen durch kontinuierliche Umsetzung von Wasserstoff und Sauerstoff an Katalysatoren, die als aktive Komponente vornehmlich Elemente der 8. Nebengruppe enthalten, wobei diese Lösungen gegebenenfalls zusätzlich Stabilisatoren enthalten können
b) Inaktivierung des Stabilisators mit Hilfe eines Ionenaustauschers
c) Umsetzung des Olefins mit besagter alkoholischer oder wäßrig-alkoholischer verdünnter Wasserstoffperoxidlösung
d) Abtrennung des epoxidierten Olefins
e) gegebenenfalls Freisetzung des Stabilisators in besagter alkoholischer oder wäßrig-alkoholischer verdünnter Wasserstoffperoxidlösung
f) Rückführung besagter alkoholischer oder wäßrig-alkoholischer verdünnter Wasserstoffperoxidlösung in das Herstellverfahren a)
umfaßt, wobei die besagte alkoholische oder wäßrig-alkoholische verdünnte Wasserstoffperoxidlösung zwischen den einzelnen Teilschritten nicht isoliert wird.

Das erfindungsgemäße Verfahren kann auf alle Olefine angewendet werden. Bevorzugt sind ungesättigte Kohlenwasserstoffe im Bereich von einem bis zwölf Kohlenstoffatomen, insbesondere Ethylen, Propylen, 1-Buten, 2-Buten, Butadien, Pentene, Hexene, Isopren, Oktene und Cyclohexene.

Die alkoholischen oder wäßrig-alkoholischen verdünnten Wasserstoffperoxidlösungen haben gewöhnlich einen Gehalt im Bereich von 0,5 bis 15 Gew.-% H₂O₂, insbesondere 2 bis 7 Gew.-%.

Als Reaktionsmedium während der Wasserstoffperoxidherstellung eignen sich Alkohole oder Wasser oder Gemische aus diesen.

Als Alkohole kommen sämtliche dem Fachmann bekannten Alkohole in Frage, wie Methanol, Ethanol, Propanol, i-Propanol, Butanol, Pentanol, Hexanol, Heptanol und Oktanol. Selbstverständlich können diese Alkohole auch verzweigt vorliegen, wie i-Butanol, s-Butanol und t-Butanol. Als bevorzugter Alkohol wird Methanol eingesetzt. Das Wasser kann selbstverständlich auch entsalzt oder destilliert sein. Die Durchführung der Wasserstoffperoxidsynthese erfolgt vorzugsweise bei geflutetem Reaktor.

Als Katalysator eigenen sich sämtliche dem Fachmann bekannte Katalysatoren zur kontinuierlichen Umsetzung von Wasserstoff und Sauerstoff zu Wasserstoffperoxid. Bevorzugt werden Katalysatoren, die als aktive Komponente vornehmlich Elemente der 8. Nebengruppe enthalten, insbesondere Palladium. Bevorzugt werden die Katalysatoren in Form von Katalysatorformkörpern eingesetzt. Katalysatorformkörper sind Katalysatoren, bei denen sich die aktive Katalysatorkomponente auf der Oberfläche von Trägern, insbesondere speziell geformten Trägern befindet, wie Raschig-Ringe, Satteikörpern, Drahtspiralen oder Maschendrahtringe. Weitere Beispiele finden sich in Römpp-Chemie-Lexikon, 9. Auflage, Seite 1453 ff. Weiterhin bevorzugt werden Katalysatormonolithe eingesetzt. Katalysatormonolithe sind in der Regel aus Geweben, Gestricken, Folien, Streckmetallen und/oder Blechen aufgebaut. Weiterhin geeignet sind offenzellige Schäume, wie Polyurethan-, Keramik- oder Melaminharzschäume. Auf diesen Katalysatormontolithen wird die aktive Katalysatorkomponente aufgebracht. Gegebenfalls enthalten die Katalysatoren Beimengungen weiterer Metalle, wie Platin, Rhodium, Iridium, Kupfer, Silber und/oder Gold um die katalytische Aktivität zu steigern. Das Verhältnis Palladium zu Beimengung liegt im Bereich von 100 zu 1 bis 1 zu 10, insbesondere 10 zu 1 bis 1 zu 1. Der Gehalt an Palladium und Beimengungen liegt in der Regel im Bereich von 5·10⁻⁴ bis 1 Gew.-%, insbesondere 10⁻³ bis 0,15 Gew.-%. Wegen weiterer Details wird auf DE-196 42 770 verwiesen, das ein bevorzugtes Verfahren zur Herstellung alkoholischen oder wäßrig-alkoholischen verdünnten Wasserstoffperoxidlösungen offenbart.

Es eignen sich jedoch auch die in DE-26 15 625, DE-26 55 920; US 4 279 883, US 4 661 337, US 4 336 239, US 4 379 778; US 4 389 390, EP-117 306, US 4 889 705, US 4681 751, US 4 772 458, US 4 240 933, US 4 832 938, WO-92/04 277, US 5 169 618, EP-579 109, EP-623 552 und beschriebenen Verfahren.

Üblicherweise enthalten die alkoholischen oder wäßrig-alkoholischen verdünnten Wasserstoffperoxidlösungen Stabilisatoren, die die Zersetzung des gebildeten Wasserstoffperoxids verhindern. Dies sind üblicherweise Mineralsäuren, wie Salzsäure, Schwefelsäure, Phosphorsäure, bevorzugt ist Salzsäure und Schwefelsäure. Diese werden üblicherweise in Mengen im Bereich von 10⁻⁴-10⁻¹ Mol/l, bevorzugt 5-25·10⁻³ Mol/l eingesetzt.

Gegebenfalls können weitere Stabilisatoren vorhanden sein, wie die in US 4 889 705 und US 4 681 751. Insbesonders vorteilhaft sind Salze wie Alkalibromide, Alkalichloride, Alkaliphosphate und Alkalisulfate, ganz besonders Natriumchlorid, Natriumbromid, Kaliumchlorid und Kaliumbromid. Diese werden üblicherweise in Mengen im Bereich von 0,5 bis 10 mmol/l, bevorzugt 0,5 bis 2 mmol/l eingesetzt.

Die zugesetzten Mengen an Säuren erweisen sich zwar für die Stabilisierung von Wassserstoffperoxid als günstig, führen aber bei der in der zweiten Stufe durchgeführten Epoxidation zu drastischen Selektivitätsminderungen bezüglich des epoxidierten Olefins. In Gegenwart von Säuren und Wasser hydrolysieren die gebildeten Epoxide zu den entsprechenden Glykolen und oligomeren Ethern. Aus diesem Grunde muß die Säurekonzentration und die Bromidionenkonzentration sehr klein gehalten werden (< 0,5 Gew.-% Säuregehalt).

Vorteilhaft werden daher im erfindungsgemäßen integrierten Verfahren die verdünnten Wasserstoffperoxidlösungen vor der Umsetzung mit Olefin pH-neutral, schwach sauer oder schwach basisch eingestellt. Bevorzugt wird ein pH-Wert im Bereich von 4 bis 9, insbesondere 5 bis 8, ganz besonders 5,5 bis 7,5. Dies erfolgt vorzugsweise mittels eines geeigneten Ionenaustauschers, jedoch sind auch andere dem Fachmann bekannten Methoden zur Neutralisation der verdünnten Wasserstoffperoxid-Lösungen geeignet.

Geeignete Ionenaustauscher bestehen z. B. aus vernetzten Polymeren. Diese Trägermaterialien können in heterogen katalysierten organischen Reaktionen sehr vielseitig eingesetzt werden. Ihre Flexibilität für strukturelle und funktionelle Veränderungen ermöglichen die Fixierung von nahezu allen katalytisch aktiven Komponenten. Starksaure Polymere, z. B. mit Sulfonsäuregruppen funktionalisiert, können oft in katalytischen Reaktionen Mineralsäuren ersetzen. Diese sauren Polymeren lassen sich mit Basen reversibel in die neutralisierte Alkaliform überführen. Weiterhin geeignet sind die in Ullmann's Encyclopedia of Industrial Chemistry, 5. Ed., (1989), A 14, S. 393-411 beschriebenen lonenaustauscher.

Es war überraschend, daß stationäre Propenoxidselektivitäten > 85 % erreicht werden, wenn die verdünnten, mineralsäurenhaltigen Wasserstoffperoxidlösungen vor der Umsetzung mit Olefin auf die beschriebene Weise über Kationenaustauscher vollständig oder teilweise neutralisiert werden.

Die Umsetzung des Olefins mit besagter alkoholischer oder wäßrig-alkoholischer verdünnter Wasserstoffperoxidlösung erfolgt bevorzugt nach dem in EP-A1-100 119 beschriebenen Verfahren. Hierbei werden epoxidierte Olefine aus Olefinen und alkoholischen oder wäßrig-alkoholischen verdünnten Wasserstoffperoxidlösungen in Gegenwart eines synthetischen Titan-Atome enthaltenden Zeoliths der allgemeinen Formel xTiO₂-(1-x)SiO₂, wobei x im Bereich von 0,0001 bis 0,04 liegt erhalten. Vorteilhaft ist es, dieses Verfahren durch die speziellen Ausgestaltungen von EP-A1-200 260, EP-A1-230 949, DE-A1-196 23 608 und DE-A1-196 23 611 zu verbessern, die hier ausdrücklich genannt werden. Selbstverständlich ist es jedoch möglich auch andere Verfahren zur Herstellung von epoxidierten Olefinen aus Olefinen und alkoholischen oder wäßrig-alkoholischen verdünnten Wasserstoffperoxidlösungen in Gegenwart eines synthetischen Titan-Atome enthaltenden Zeoliths der allgemeinen Formel xTiO₂·(1-x)SiO₂, wobei x im Bereich von 0,0001 bis 0,04 liegt, im erfindungsgemäßen integrierten Verfahren einzusetzen.

Es kann auch vorteilhaft sein, einen Teil oder alle Titanatome durch Vanadiumatome zu ersetzen. Weiterhin ist es vorteilhaft, die sauren Oberflächenzentren der synthetischen Titan-Atome enthaltenden Zeolithe der allgemeinen Formel xTiO₂·(1-x)SiO₂, wobei x im Bereich von 0,0001 bis 0,04 liegt, zu neutralisieren (z. B. mit Alkalisalzen wie Natriumacetat).

Die Abtrennung des gebildeten epoxidierten Olefins erfolgt auf üblichem Wege, z. B. durch Destillation gegebenenfalls unter vermindertem Druck.

Zur H₂O₂-Bildung muß das Reaktionsmedium möglichst protonenreich, zur Epoxidation möglichst protonenarm sein Für eine Rückführung der ursprünglich eingesetzten alkoholischen oder wäßrig-alkoholischen verdünnten Wasserstoffperoxidlösung mit nun verringerter Wasserstoffperoxidkonzentration in das besagte Verfahren zur Herstellung alkoholischer oder wäßrig-alkoholischer verdünnter Wasserstoffperoxidlösung kann es nach Abtrennung von Propenoxid vorteilhaft sein, den gegebenfalls inaktivierten oder neutralisierten Stabilisator wieder freizusetzen. Dies geschieht vorteilhaft durch Zugabe oder Durchströmung eines entsprechenden Ionenaustauschers zur abgereicherten Wasserstoffperoxidlösung. Besonders vorteilhaft ist es, den zuvor eingesetzten Kationenaustauscher unter gegebenfalls veränderten Reaktionsparametern wieder auf diese Weise zu regenerieren. Selbstverständlich kann auf dieser Stufe auch zusätzlicher Stabilisator zugegeben, bzw. verbrauchter oder verlorener Stabilisator ersetzt werden.

Geeignete Ionenaustauscher sind organische und/oder anorganische Polymere, die z.B. mit Sulfonsäuregruppen funktionalisiert sind (z.B. Ionenaustauscher von Bayer AG, D (z.B. Lewatit®, Bayer Catalyst® K 1131 oder K 2431) oder von Rohm and Haas, USA (z. B. Amberlist® 36 W, Amberlist® 3 WET, Duolite® ARC 9652). Weiterhin geeignet sind die in Ullmann's Encyclopedia of Industrial Chemistry, 5. Ed., (1989), A 14, S. 393-411 beschriebenen Ionenaustauscher.

Schließlich wird die abgereicherte Wasserstoffperoxidlösung wieder in das Herstellverfahren für alkoholische oder wäßrig-alkoholische verdünnte Wasserstoffperoxidlösung (Schritt a)) zurückgeführt.

Die auf diese Weise herstellbare epoxidierten Olefine sind insbesondere Ethenoxid, Propenoxid.

Die auf diese Weise herstellbaren epoxidierten Olefine zeichnen sich durch hohe Reinheit aus. Das Gesamtverfahren ist ökologisch und ökonomisch sehr sparsam und nachhaltig.

Die auf diese Weise herstellbare epoxidierten Olefine sind für sämtliche dem Fachmann bekannten Verwendungszwecke einsetzbar, wie Herstellung von Polyetherpolyolen, Glykolen (Schmier und Frostschutzmittel), Propenoxid-Derivaten, und zur Sterilisation von Gegenständen im medizinischen Bereich.

### Beispiele

### Beispiel 1

### Herstellung von Katalysatormonolithen mit Katalysatorträgergeweben aus V4A

Analog DE 196 42 770 A1 wird ein zylindrischer V4A-Monolith aus gewellten und glatten V4A-Netzen hergestellt (Material: 1.4571; Maschenweite: 200 µm; Drahtdurchmesser: 150 µm).

Dieser monolithische Träger wurde nach den üblichen Methoden von Fett mit Lösungsmitteln befreit und in 80 ml destilliertem Wasser vorgelegt, 5 ml einer 10%igen [Pd(NH₃)₄](NO₃)₂-Lösung hinzugegeben und bei 80°C 24 h gerührt. Der Monolith wird von der Flüssigphase abgetrennt, 4 mal mit je 100 ml VE-Wasser gewaschen, 2 h bei 110°C getrocknet und anschließend unter Stickstoff 2 h bei 200°C reduziert (Autoreduktion durch thermische Zersetzung des Aminkomplexes unter Bildung von Ammoniak).

### Beispiel 2

### a) Herstellung von Wasserstoffperoxid

In einem 400 ml V4A-Rührautoklaven mit Gasrührer, Wärmeträgerthermostatisierung, Druckhalterung bis 70 bar und zentralem Meßwerterfassungssystem wurde der monolithische Pd-Katalysator aus Beispiel 1 um die Rührerachse zentriert eingebaut. Im Reaktorboden befinden sich Zuleitungen für Wasserstoff, Sauerstoff und das Reaktionsmedium. Im Reaktordeckel befindet sich eine Ableitung, aus der das Flüssig/Gasgemisch kontinuierlich entnommen wird.

Das Reaktionsmedium bestand aus Methanol, dem 0,3 Gew.-% Schwefelsäure, 0,03 Gew.-% Phosphorsäure und 5 ppm Bromid (in Form von Natriumbromid) zugeführt wurde. Mit einem Volumenstrom von 100 g/h Reaktionsmedium wird der Reaktor zusammen mit einem konstanten Stickstoffstrom von 60 l/h bei konstant 20 bar durchflossen. Zum Start der Reaktion wird der Stickstoffstrom durch den gleichen Gesamtvolumenstrom aus Sauerstoff und Wasserstoff im Volumenverhältnis 92 : 8 ersetzt.

Die Wasserstoffperoxidkonzentration der erhaltenen Methanol/Wasser-Lösungen betragen 2-6 Gew.-% Wasserstoffperoxid. Die Lösung aus 2a) wird direkt in Beispiel 2 b) eingesetzt.

### b) Neutralisation der H₂SO₄/H₃PO₄/H₂O₂-methanolischen Lösung aus Beispiel 2a)

Vorbehandlung des Ionenaustauschers: 100 g Lewatit S 100 (Firma Bayer) werden in 300 g 10 %iger NaOH für 2 h gerührt, dann filtriert und portionsweise mit insgesamt 1,5 l Wasser gewaschen, bis das Waschwasser pH neutral ist. Der Kationenaustauscher in der Natriumform wird zum Austausch Wasser-Methanol 3 x in je 100 g Methanol je 30 min gerührt.

Der so vorbehandelte Ionenaustauscher wurde mit 100 g Methanol in eine Chromatographiesäule (3 cm Durchmesser, 30 cm lang; 12 cm Höhe der Lewatitschüttung) überführt.

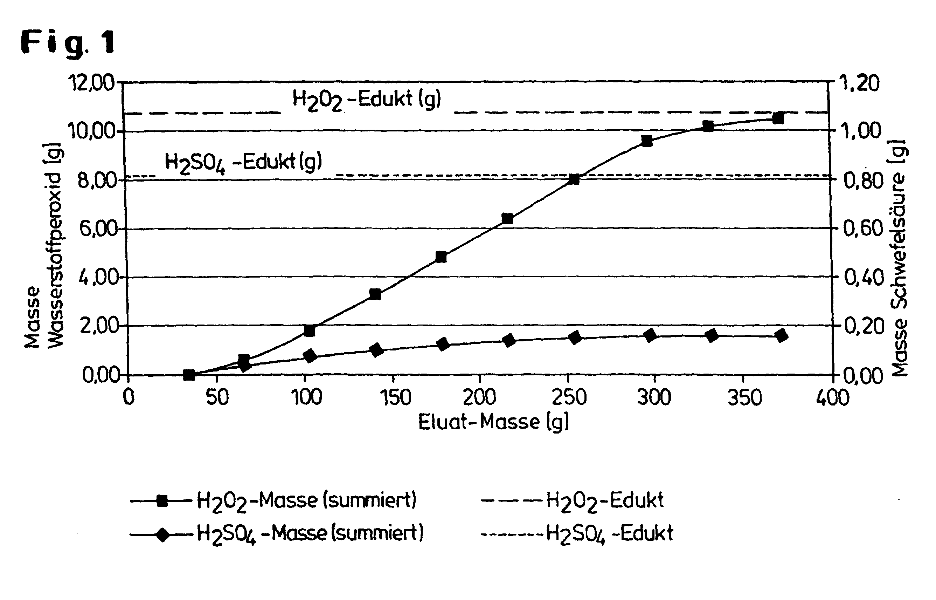

251 g der H₂SO₄/H₃PO₄/H₂O₂-methanolischen Lösung werden in 50 min durch den vorbehandelten Ionenaustauscher gegeben, mit 150 ml Methanol nachgespült und das Eluat in 10 Fraktionen von je ca. 50 ml aufgefangen. Im Edukt und in den Fraktionen wird der Säuregehalt und der H₂O₂-Gehalt titrimetrisch bestimmt. Das Wasserstoffperoxid verläßt vollständig, d.h. ohne Zersetzung, die Chromatographiesäule. Die enthaltene Schwefelsäure und Phosphorsäure kann durch einen einmaligen Durchgang durch die Säule zu ca. 75-85 % neutralisiert werden (Abb. 1).

Säuretitration: ca. 10 g Probe werden eingewogen, mit Wasser auf ca. 150 ml aufgefüllt und nach Zugabe von 5 Tropfen 1 %iger Phenolphthaleinlösung gegen 0,1 n NaOH (0 4 g/l) titriert (Blindwert < 1 Tropfen).

H202-Titration: 1 g Probe wird mit 60 ml Schwefelsäure (1 mol/l) versetzt, mit Wasser auf 170 ml aufgefüllt und gegen 0,1 n KmnO4 (3,16 g/l) titriert (Blindwert < 1 Tropfen). Die Lösung aus Beispiel 2 b) wird direkt in Beispiel 2 c) eingesetzt.

### c) Herstellung von Propenoxid

In einem 11-Rührautoklaven mit einem magnetischen Rührer, Wasserthermostatisierung, Thermoelement und Zuleitungen im Reaktorboden für die frisch hergestellte teilneutralisierte Wasserstoffperoxidlösung aus 2 b) (ca. 3 bis 5 %ig) und für gasförmiges Propylen. In einer typischen Herstellungsmethode werden in den mit Stickstoff gespülten Reaktor bei 45°C 500 g der verdünnten Wasserstoffperoxidlösung vorgelegt, 2 g synthetisches Titansilikalit mit Titangehalten von 2,8 Gew,-% wie in EP-A1-100 119 beschrieben suspendiert und unter heftigen Rühren gasförmiges Propylen bis 4 bar eindosiert. Weiteres Propylen wird in dem Maße dosiert, wie es abreagiert; der Druck von 4 bar bleibt also konstant. Unter diesen Bedingungen verläuft die Epoxidation zum Propenoxid sehr schnell und selektiv. Nach 70 min ist mehr als 95 % des Wasserstoffperoxides verbraucht und es wird eine Propenoxid-Selektivität von 85 bis 92 % gefunden (Abb. 1).

### d) Freisetzung des Stabilisators aus 2 b)

Die abgereicherte Lösung aus 2 c) wurde nach destillativer Abtrennung von Propenoxid in umgekehrter Richtung durch die in 2 b) verwendete Chromatographiesäule geleitet.

Die neutralisierte Säure wurde zu über 70 bis 80 % wieder in die saure Form überführt. Der pH-Wert betrug < 1.

## Patentansprüche

1. Verfahren zur Herstellung von epoxidierten Olefinen aus Olefinen und alkoholischen oder wäßrig-alkoholischen verdünnten Wasserstoffperoxidlösungen in Gegenwart eines synthetischen Titan-Atome enthaltenden Zeoliths der allgemeinen Formel xTiO₂·(1-x)SiO₂, wobei x im Bereich von 0,0001 bis 0,04 liegt, **dadurch gekennzeichnet, daß** das Verfahren die Schritte
a) Herstellung der alkoholischen oder wäßrig-alkoholischen verdünnten Wasserstoffperoxidlösungen durch kontinuierliche Umsetzung von Wasserstoff und Sauerstoff an Katalysatoren, die als aktive Komponente vornehmlich Elemente der 8. Nebengruppe enthalten, wobei diese Lösungen zusätzlich Stabilisatoren enthalten
b) Inaktivierung des Stabilisators mit Hilfe eines Ionenaustauschers
c) Umsetzung des Olefins mit besagter alkoholischer oder wäßrig-alkoholischer verdünnter Wasserstoffperoxidlösung
d) Abtrennung des epoxidierten Olefins
e) gegebenenfalls Freisetzung des Stabilisators in besagter alkoholischer oder wäßrig-alkoholischer verdünnter Wasserstoffperoxidlösung
f) Rückführung besagter alkoholischer oder wäßrig-alkoholischer verdünnter Wasserstoffperoxidlösung in das Herstellverfahren a)
enthält, wobei die besagte alkoholische oder wäßrig-alkoholische verdünnte Wasserstoffperoxidlösung zwischen den einzelnen Teilschritten nicht isoliert wird.

2. Verfahren gemäß Anspruch 1, **dadurch gekennzeichnet, daß** als Katalysator im Schritt a) Palladium oder Palladium-Metallgemische eingesetzt werden.

3. Verfahren gemäß einem der Ansprüche 1 bis 2, **dadurch gekennzeichnet, daß** besagte Lösungen in Schritt a) Mineralsäuren als Stabilisatoren enthalten.

4. Verfahren gemäß einem der Ansprüche 1 bis 3, **dadurch gekennzeichnet, daß** die Freisetzung des Stabilisators mit Hilfe eines Ionenaustauschers erfolgt.

5. Verfahren gemäß einem der Ansprüche 1 bis 4, **dadurch gekennzeichnet, daß** als Olefin Propylen verwendet wird.

## Claims

1. Process for the preparation of epoxidized olefins from olefins and dilute alcoholic or aqueous-alcoholic hydrogen peroxide solutions in the presence of a synthetic zeolite containing titanium atoms of the general formula xTiO₂·(1-x)SiO₂, where x ranges from 0.0001 to 0.04, **characterized in that** the process comprises the following steps:
a) preparation of the dilute alcoholic or aqueous-alcoholic hydrogen peroxide solutions by the continuous reaction of hydrogen and oxygen on catalysts containing mainly elements of subgroup 8 as the active component, said solutions additionally containing stabilizers,
b) inactivation of the stabilizer with the aid of an ion exchanger,
c) reaction of the olefin with said dilute alcoholic or aqueous-alcoholic hydrogen peroxide solution,
d) separation of the epoxidized olefin,
e) optionally liberation of the stabilizer in said dilute alcoholic or aqueous-alcoholic hydrogen peroxide solution, and
f) recycling of said dilute alcoholic or aqueous-alcoholic hydrogen peroxide solution into preparative process a),
said dilute alcoholic or aqueous-alcoholic hydrogen peroxide solution not being isolated between the individual partial steps.

2. Process according to Claim 1, **characterized in that** palladium or palladium/metal mixtures are used as the catalyst in step a).

3. Process according to Claim 1 or 2, **characterized in that** said solutions in step a) contain mineral acids as stabilizers.

4. Process according to one of Claims 1 to 3, **characterized in that** the stabilizer is liberated with the aid of an ion exchanger.

5. Process according to one of Claims 1 to 4, **characterized in that** propylene is used as the olefin.

## Revendications

1. Procédé pour la préparation d'oléfines époxydées à partir d'oléfines et de solutions alcooliques ou hydro-alcooliques diluées de peroxyde d'hydrogène en présence d'une zéolite synthétique contenant des atomes de titane et répondant à la formule générale xTiO₂,(1-x)SiO₂, dans laquelle x est un nombre allant de 0,0001 à 0,04, **caractérisé en ce qu'**il comprend les stades opératoires suivants :
a) préparation des solutions alcooliques ou hydro-alcooliques diluées de peroxyde d'hydrogène par réaction continue de l'hydrogène et de l'oxygène sur des catalyseurs contenant en tant que composants actifs principalement des éléments du 8^{ème} sous-groupe, ces solutions contenant en outre des stabilisants,
b) désactivation du stabilisant à l'aide d'un échangeur d'ions,
c) réaction de l'oléfine avec la solution alcoolique ou hydroalcoolique diluée de peroxyde d'hydrogène,
d) séparation de l'oléfine époxydée,
e) le cas échéant, libération du stabilisant de la solution alcooolique ou hydro-alcoolique diluée de peroxyde d'hydrogène,
f) recyclage de la solution alcoolique ou hydroalcoolique diluée de peroxyde d'hydrogène au stade préparation a),
et **en ce que** la solution alcoolique ou hydro-alcoolique diluée de peroxyde d'hydrogène n'est pas isolée entre les diverses stades opératoires.

2. Procédé selon revendication 1 **caractérisé en ce que** le catalyseur utilisé au stade opératoire a) est le palladium ou un mélange palladium-autres métaux.

3. Procédé selon l'une des revendications 1 à 2 **caractérisé en ce que** les solutions de peroxyde d'hydrogène au stade opératoire a), contiennent des acides minéraux en tant que stabilisants,

4. Procédé selon l'une des revendications 1 à 3 **caractérisé en ce que** la libération du stabilisant est réalisée à l'aide d'un échangeur d'ions.

5. Procédé selon l'une des revendications 1 à 4 **caractérisé en ce que** l'oléfine mise en oeuvre est le propylène.
